(19) Europäisches Patentamt
European Patent Office
Office européen des brevets

(11) Veröffentlichungsnummer: **0 073 995**
**B1**

(12) # EUROPÄISCHE PATENTSCHRIFT

(45) Veröffentlichungstag der Patentschrift: **20.05.87**

(51) Int. Cl.⁴: **C 08 F 2/50,** G 03 C 1/68, A 61 K 6/08

(21) Anmeldenummer: **82107677.5**

(22) Anmeldetag: **23.08.82**

(54) **Photopolymerisierbare Massen, deren Verwendung für zahnärztliche Zwecke, sowie Verfahren zur Herstellung von Zahnersatzteilen, Zahnfüllungen und Überzügen.**

(30) Priorität: **04.09.81 DE 3135113**

(43) Veröffentlichungstag der Anmeldung: **16.03.83 Patentblatt 83/11**

(45) Bekanntmachung des Hinweises auf die Patenterteilung: **20.05.87 Patentblatt 87/21**

(84) Benannte Vertragsstaaten: **AT BE CH DE FR GB IT LI LU NL SE**

(56) Entgegenhaltungen:
**GB-A-1 547 919**
**US-A-3 756 827**
**US-A-4 089 763**

(73) Patentinhaber: **BAYER AG, Konzernverwaltung RP Patentabteilung, D-5090 Leverkusen 1 Bayerwerk (DE)**

(72) Erfinder: **Schmitz- Josten, Robert, Dr., Gerstenkamp 6, D-5000 Köln 80 (DE)**
Erfinder: **Süling, Carlhans, Dr., Carl- Leverkus-Strasse 10, D-5068 Odenthal (DE)**
Erfinder: **Podszun, Wolfgang, Dr., Wolfskaul 4, D-5000 Köln 80 (DE)**
Erfinder: **Bömer, Bruno, Dr., Gustav- Freytag-Strasse 2, D-5090 Leverkusen 1 (DE)**
Erfinder: **Borgardt, Manfred, Dr., Roediger-Strasse 17, D-5600 Wuppertal 1 (DE)**
Erfinder: **Walkowiak, Michael, Dr., Albertus-Magnus- Strasse 10, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

EP 0 073 995 B1

**Beschreibung**

Die Erfindung betrifft photopolymerisierbare Massen, enthaltend mindestens ein ethylenisch ungesättigtes polymerisierbares Monomer und ein lichtempfindliches Katalysatorsystem. Die erfindungsgemäßen Massen eignen sich insbesondere zur Herstellung von gefüllten und ungefüllten, pastenförmigen und flüssigen Dentalmassen, von Zahnersatzteilen, Zahnreparaturmaterialien und Zahnfüllmassen sowie von Überzugs- und Klebemassen für zahnärztliche Zwecke.

Es ist bekannt, mit Hilfe von Strahlungsenergie, wie Ultraviolett- und/oder sichtbarem Licht die Polymerisation von ethylenisch ungesättigten, radikalisch polymerisierbaren Monomeren zu induzieren. Hierzu werden meist Photoinitiatoren verwendet, welche entweder durch direkte Lichtabsorption oder durch Energieübertragung von einem auf photochemischem Wege angeregten Sensibilisator in einen chemisch reaktive angeregten Zustand übergehen. Die Bildung von die Polymerisation auslösenden Radikalen erfolgt entweder durch Fragmentierung der angeregten Photoinitiatoren oder durch deren Reaktion mit anderen Molekülen, z.B. Wasserstoff- oder Elektronendonatoren, wobei aus diesen anderen Molekülen die polymerisationsauslösenden Radikale gebildet werden. Bei der photochemisch initiierten radikalischen Polymerisation ist hinsichtlich der Lichtabsorption zu beobachten, daß die Absorptionsbanden der Sensibilisatoren bzw. Initiatoren möglichst gut mit den Hauptemissionsbanden der verwendeten Strahlungsquellen übereinstimmen. Von Bedeutung ist auch der Extinktionskoeffizient der Sensibilisatoren und Photoinitiatoren. Zu hohe Extinktionskoeffizienten bzw. zu hohe Konzentrationen der Sensibilisatoren bzw. Photoinitiatoren oder anderer Zusätze bewirken nur eine oberflächliche Polymerisation der Monomeren mit den bekannten Nachteilen.

Zu den an sich bekannten photofragmentierbaren Initiatoren gehören beispielsweise Peroxide, Azoverbindungen, α-Acyloximester, wie 1-Phenyl-1,2-propandion-2-o-benzoyl-oxim, ferner Disulfide, Phenylglyoxalate, α-Halogenketone, wie Trichloracetophenon, Halogenmethylgruppen tragende Aromaten und Arylsulfochloride. Besondere Bedeutung für die Polymerisation mit ultraviolettem Licht haben Carbonylverbindungen erlangt, z.B. Acyloine, Benzoin und seine Derivate (vgl. hierzu beispielsweise US-PS 2 367 661; H.G. Heine, H.J. Rosenkranz und H. Rudolph, Angewandte Chemie 84, 1032 (1972). Sehr wirksam sind auch die in der US-PS 3 715 293 beschriebenen Alkoxy-acetophenonderivate, wie 2,2-Diethoxyacetophenon und Benzildimethylketal sowie die in der DE-OS 2 808 459 beschriebenen Hydroxyalkylphenone.

Zur zweiten Gruppe von Photoinitiatoren, welche nur dann Photopolymerisationen auslösen, wenn gleichzeitig synergistisch wirkende Wasserstoffdonatoren oder andere radikalbildende Stoffe ("radical generating agents") zugegen sind, gehören die aromatischen Monoketone vom Benzophenontyp, wie Benzophenon, Fluorenon, Xanthon, Thioxanthon, Dibenzosuberon, aber auch die polynuclearen Chinone, wie Anthrachinon (US 2 989 455), gewisse oxydierend wirkende Farbstoffe und 1,2-Diketone aus der aromatischen, cycloaliphatischen und aliphatischen Reihe, z.B. Phenanthranchinon, 2,3-Bornandion (US-PS 2 951 758), Benzil (US-PS 2 367 660) und Diacetyl oder Dipivaloyl.

Zu den erwähnten synergistisch wirkenden Verbindungen, welche als Wasserstoffdonatoren, Kettenüberträger (US-PS 3 046 127), reduzierend wirkende Verbindungen oder generell als Photoaktivatoren (Europ. Offenlegungsschrift 2625) bezeichnet werden, gehören gewisse Ether, z.B. Tetrahydrofuran, Alkohole, Mercaptane, Thioether, Alkylphosphine, N-Allylverbindungen, wie Allylthioharnstoff und insbesondere Alkylgruppen-enthaltende Amine, welche in α-Stellung zum Stickstoff eine CH-Gruppe tragen. Vgl. hierzu beispielsweise M.R. Sandner et al., Journal of Polymer Science, Polymer Chemistry Edition 10 (1972), S. 3173-3181, ferner die US-PS 3 759 807. Beispiele für die Kombination von α-Diketonen mit reduzierend wirkenden Aminen sind in den US-PSS 3 450 613, 4 071 424 sowie in der DE-OS 2 003 132 (Beispiel 34) enthalten.

Es sind auch Aminocarbonylverbindungen mit der Gruppierung

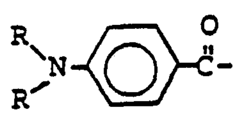

als Photoinitiatoren beschrieben worden.

Der bekannteste Vertreter dieser Gruppe ist das 4,4'-Bis-(dimethylamino)-benzophenon oder Michler's Keton, welches für sich alleine, aber auch in Kombination mit Benzophenon und anderen Carbonylverbindungen und Peroxiden Photopolymerisationen auszulösen vermag (vgl. DE-PS 760 351, US-PSS 3 427 161, 3 718 473). Bei seiner Verwendung treten jedoch starke Verfärbungen der Polymerisationsprodukte auf.

In der DE-OS 2 458 345, Europ. PS 2625 und der GB-PS 1 547 919 werden die para- und ortho-Dialkylaminobenzoesäureester als weniger stark verfärbende Reduktionsmittel (Photoaktivatoren) in Kombination mit Benzophenon oder 2-Chlorthioxanthon vorgeschlagen, doch verfärben sich auch die so hergestellten Photopolymerisate bei längerer Belichtung noch deutlich.

Es ist auch bekannt, gefüllte und ungefüllte Dentalmassen durch Photopolymerisation herzustellen. Die DE-PS 760 351 beschreibt die Photopolymerisation von plastischen Gemischen aus monomeren und polymeren Methacrylsäure-Estern in Gegenwart von Benzoylperoxid und Michler's Keton durch die Einwirkung von UV-Licht und/oder Wärmestrahlung.

In der GB-PS 569 974 werden photopolymerisierbare Dentalmassen aus Monomer-Polymergemischen beschrieben, welche als Photoinitiatoren Acyloine, Benzoin oder Polyketaldonylverbindungen der Formel R-$(CO)_n$-$R^1$ enthalten, z.B. Diacetyl, Benzil, Phenylglyoxal, 1,2-Cyclohexandion oder Diphenyltriketon. In den US-PSS 3 629 187 und 3 709 866 werden UV-härtbare Dentalmassen beschrieben, welche anorganische Füllstoffe enthalten. Die US-PSS 4 089 763 und 4 110 184 sowie die DE-OS 2 419 887 beschreiben Zahnfüllmaterialien, welche Urethanacrylate, anorganische Füllstoffe, α-Diketone und ungesättigte Amine, wie Dimethylaminoethylmethacrylat oder andere reduzierend wirkende Verbindungen enthalten. Die Europ. PS 12 535 beschreibt spezielle Kombinationen aus Urethanacrylaten, polymerisierbaren Verdünnern, α-Diketonen und organischen Aminen, welche als photopolymerisierbare Fissurenversiegler, Haftvermittler oder als orthodontische Klebstoffe verwendet werden können.

Die DE-OS 2 751 057 und die US-PS 4 192 795 beschreiben photopolymerisierbare, röntgenopake Dental-Reparaturmaterialien, welche als Photoinitiatoren α-Diketone (Benzil) und als Photoaktivatoren Alkylamine, wie Dimethylbenzylamin oder Dimethyl-paratoluidin enthalten.

Schließlich wird in der DE-OS 28 56 550 ein photopolymerisierbares, opakes Zahnfüllmaterial beschrieben, welches 1 bis 20 % Calciumfluorid enthält. Als Photoinitiatoren werden Benzildimethylketal bzw. α-Methylbenzoinmethylether in Kombination mit Phosphiten oder das System Phenanthrenchinon-Dimethylaminoethylmethacrylat (DMAEM) verwendet. Das DMAEM ist jedoch als stark gewebereizend bekannt.

Es wurden photopolymerisierbare Massen, enthaltend

a) mindestens ein ethylenisch ungesättigtes, photopolymerisierbares Monomer,

b) mindestens einen Photoinitiator aus der Gruppe der organischen Mono- und Dicarbonylverbindungen und

c) mindestens ein aromatisches Amin, das in α-Stellung zum Stickstoff eine CH-Gruppe aufweist, als Photoaktivator, und gegebenenfalls

d) mindestens einen feinteiligen anorganischen und/oder organischen Füllstoff,

gefunden, die dadurch gekennzeichnet sind, daß die Photoaktivatoren Alkylaminoarylsulfonylverbindungen der allgemeinen Formel

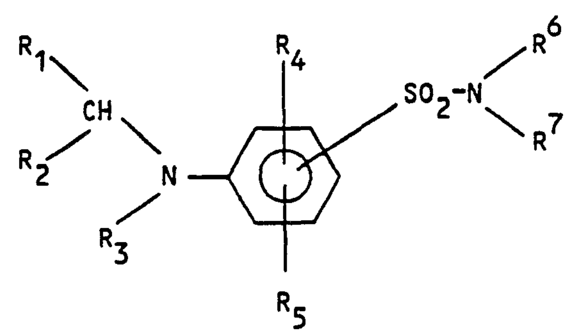

in welcher

$R^1$ und $R^2$ gleich oder verschieden sind und Wasserstoff, Methyl, Vinyl oder Phenyl bedeuten,

$R^3$ Wasserstoff, Methyl und Ethyl bedeutet,

$R^4$ und $R^5$ gleich oder verschieden sind und Wasserstoff, Halogen oder Methyl bedeuten oder orthoständiges $R^4$ und $R^5$ Teile eines annellierten aromatischen Sechsrings sind,

$R^6$ und $R^7$ gleich oder verschieden sind und Wasserstoff, Methyl, Allyl, Methallyl, Cyclohexyl, Phenyl, Benzyl, Hydroxyethyl, Hydroxypropyl, Acryloxyalkyl, Methacryloxyalkyl, 2,3-Epoxypropyl oder 1,2-Dihydroxypropyl-1-(meth-)acrylat bedeuten und wobei $R^6$ und $R^7$ zu einem Morpholin- oder Piperidinrest verbunden sein können, sind.

Gegenstand der Erfindung ist weiterhin die Verwendung der erfindungsgemäßen photopolymerisierbaren Massen für zahnärztliche Zwecke, insbesondere in einem Verfahren zur Herstellung von Zahnersatzteilen und Zahnfüllungen durch Polymerisation der Massen unter Lichteinwirkung in einer geeigneten Form.

Die in den erfindungsgemäßen Massen enthaltenen Photoinitiatoren aus der Gruppe der Mono- und Dicarbonylverbindungen sind an sich bekannt; beispielhaft seien die folgenden genannt:

Anthrachinon, 2-Ethylanthrachinon, 2-t-Butylanthrachinon, 1-Chloranthrachinon, 1,2-Benzanthrachinon, 2,3-Benzanthrachinon, Phenanthrenchinon, Benzil, Naphthil, Furil, 5,12-Naphthochinon, Acenaphtenchinon, Diacetyl, Dipivaloyl, 2,3-Bornandion, 1,1,4,4-Tetramethyl-tetralin-2,3-dion, 2,2,5,5-Tetramethyl-tetrahydro-3,4-furandion, Imidazoltrion, Isatin, Benzophenon, Benzophenon-tetracarbonsäure-derivate, Acetophenon, Propiophenon, Trichloracetophenon, Phenylcyclopropylketon, 1,3-Diphenyl-2-propanon, 1,3,5-Triacetylbenzol,

Anthron, Benzanthron, 4-Acetylbiphenyl, Benzoylbiphenyl, β-Naphthylphenylketon, Dibenzosuberon, Xanthon, Fluorenon, Thioxanthon, 2-Chlorthioxanthon, 2-Benzoylbenzophenon, Benzophenon-o-carbonsäure und deren Derivate sowie Acridon und seine Derivate.

Bevorzugt sind aus der Reihe der α-Diketone das Phenanthrenchinon und solche cycloaliphatischen α-Diketone, welche nicht zur intramolekularen Wasserstoffabstraktion befähigt sind, z.B. das 2,3-Bornandion.

Die Photoinitiatoren werden bevorzugt in Mengen von 0,001-10 Gew.-%, insbesondere 0,01-5 Gew.-%, bezogen auf das Monomer, eingesetzt. Das Molverhältnis der Photoinitiatoren zu den Photoaktivatoren der Formel (I) kann z.B. 0,01-10 betragen. Der Vorzugsbereich liegt bei 0,1-1. Die Photoinitiatoren und die Photoaktivatoren können auch als Gemische eingesetzt werden.

Erfindungsgemäß können auch weitere, nicht zur Gruppe der Mono- und Dicarbonylverbindungen gehörige Photoinitiatoren zusätzlich verwendet werden. Hierzu zählen beispielsweise photoreduzierbare Farbstoffe gemäß US-PSS 2 850 445, 3 579 339 und 3 495 987. Auch die eingangs beschriebenen photofragmentierbaren Photoinitiatoren können zugesetzt werden, um beispielsweise den verwendbaren Wellenlängenbereich der Strahlungsquellen zu erweitern. Hierzu sind z.B. das Benzildimethylketal, die Benzoinether und die in der DE-OS 2 808 459 beschriebenen Hydroxyalkylphenone geeignet. Weitere vorteilhafte Kombinationsmöglichkeiten ergeben sich durch den gemeinsamen Einsatz von α-Diketonen mit den Alkylaminoarylsulfonylverbindungen gemäß Formel (I) und Sensibilisatoren gemäß der US-PS 3 756 827. Die Verbindungen gemäß Formel (I) können auch anstelle der in der DE-PS 2 625 538 genannten Amine in Kombination mit den dort aufgeführten Mono- und Dicarbonylverbindungen verwendet werden.

Die erfindungsgemäß in Kombination mit Mono- und Dicarbonylverbindungen einzusetzenden reduzierend wirkenden Photoaktivatoren der Formel (I) enthalten als wirksame Struktureinheiten eine Aminoalkylgruppe mit mindestens einem Wasserstoffatom in α-Stellung zum Aminstickstoff, gebunden an einem mindestens eine Sulfonylgruppe $SO_2$-X tragenden aromatischen Kern. Vorzugsweise befindet sich die Aminoalkylgruppe in meta- oder para-Stellung, besonders bevorzugt in para-Stellung zur Sulfonylgruppe. Die Substutuenten $R_1$-$R_5$ und X sind an sich beliebig, doch sind $R_1$ und $R_2$ besonders bevorzugt Wasserstoff und $R_3$ Methyl. Die übrigen Substituenten dürfen jedoch den beabsichtigten Zweck nicht beeinträchtigen, d.h. die Polymerisation oder die Lichtstabilität herabsetzen. Ferner sollen die Substituenten eine ausreichende Löslichkeit der Aktivatoren in den verwendeten Monomeren gewährleisten.

Die Aktivatoren weisen als Substituenten -$SO_2$-X vorzugsweise eine Sulfongruppe und besonders bevorzugt eine Sulfonamidgruppe mit aliphatischen, cycloaliphatischen oder heterocyclischen Substituenten auf. Ein weiterer Vorzugsbereich umfaßt Alkylaminoarylsulfonamide bzw. -sulfone, welche polymerisierbare Gruppen enthalten, beispielsweise Allylgruppen, ferner (Meth-)Acrylestergruppen oder Maleinsäureestergruppen, welche leicht nach an sich bekannten Methoden in Hydroxyalkyl- oder Epoxyalkylgruppen-haltige Verbindungen der allgemeinen Formel (I) eingeführt werden können. Geeignete Zwischenprodukte hierfür sind die in der nachstehenden Tabelle 1 aufgeführten Verbindungen 1a, 7, 10 und 12. Eine weitere Möglichkeit zur Einführung von polymerisierbaren Gruppen in (I) besteht in der Umsetzung von Hydroxygruppen oder Aminogruppen tragenden Alkylaminoarylsulfonylverbindungen der Formel (I) mit ungesättigten Isocyanaten bzw. Isocyanatgruppen tragenden ungesättigten Urethanprepolymeren. Photokatalysatoren der Formel (I), bei welchen X die Gruppe N

$(CH_2-CH-OH)_2$
R

bedeutet (R = H oder $CH_3$), können mit Vorteil analog zu den DT-PSS 919 431 und 1 942 954 in Polyester einkondensiert werden. Verbindungen der Formel I, bei welchen X die Gruppe NH

$R_1$

bedeutet, können mit N-Methoxymethylacrylamid oder -methacrylamid zu ungesättigten Photoaktivatoren

$-SO_2-N-CH_2-NH-C-C=CH_2$ umgesetzt werden.
$R_1$ O R

Da solche polymerisierbaren bzw. einkondensierten Photoaktivatoren der Formel (I) in den Polymerverbund eingebaut werden, sind sie besonders für mit dem menschlichen Körper in Kontakt kommenden photopolymerisierbare Massen geeignet, z.B. für die Verwendung als Knochenzemente, Dentalzemente, Zahnfüllmassen und Versiegelungsmassen. Infolge ihrer geringen Basizität sind sie nicht gewebereizend und dadurch anderen polymerisierbaren reduzierend wirkenden Photoaktivatoren wie z.B. dem Dimethylaminoethylmethacrylat (DMAEM) überlegen, welches als aliphatisches Amin stark gewebereizend ist.

Die Herstellung der Alkylaminosulfonylverbindungen der Formel (I) erfolgt nach an sich bekannten Methoden, wie sie beispielsweise bei Houben-Weyl, "Methoden der organischen Chemie", Georg Thieme Verlag 1955, Band IX, Seiten 227 ff und 606 ff beschrieben sind.

Viele als Vorprodukte geeignete Verbindungen sind z.B. beschrieben in E.M. Northey, "The Sulfonamides and allied compounds", Reinhard Publishing Corporation, New York 1948.

Es können beispielsweise die in der nachstehenden Tabelle 1 aufgeführten, teilweise neuen Verbindungen eingesetzt werden (siehe die Ausführungsbeispiele):

**Tabelle 1**

| Verbindung-Nr. | Formel | Fp. (°C) |
|---|---|---|
| 1 (J.A.C.S. 68, (1946), 1184, 1191) | 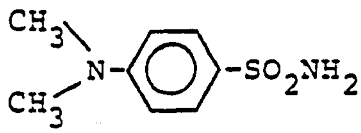 | 210-212 |
| 1a | 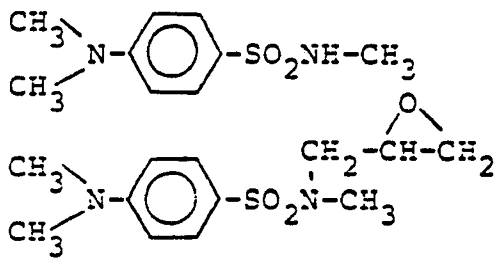 | 142 |
| 2 | | 85-86 |
| 3 | 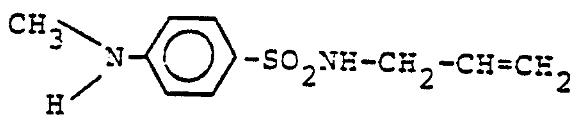 | 91-93 |
| 4 (bevorzugt) | 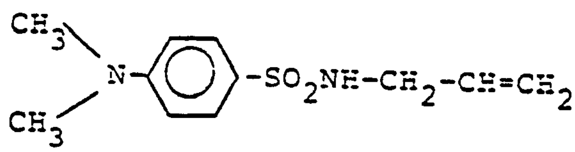 | 111-112 |
| 5 | 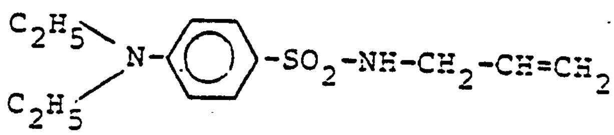 | 87-88 |
| 6 (bevorzugt) | 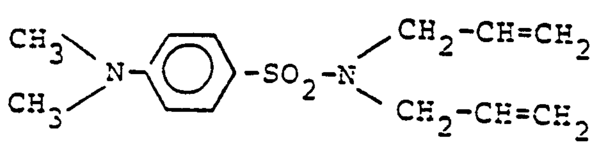 | 75 |

**Tabelle I (Fortsetzung)**

| Verbindung Nr. | Formel | Fp. (°C) |
|---|---|---|
| 7 (bevorzugt) | 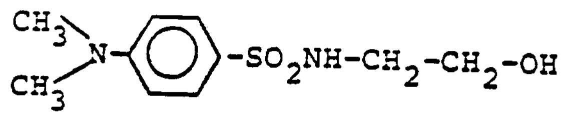 | 104-106 |
| 7a (bevorzugt) | 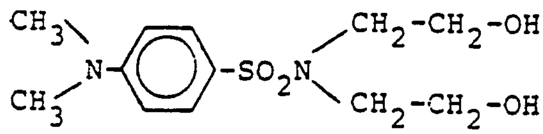 | |
| 8 (bevorzugt) | 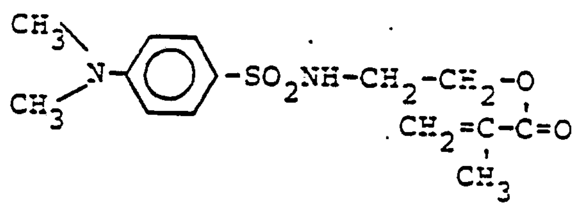 | 122 |
| 9 (bevorzugt) | 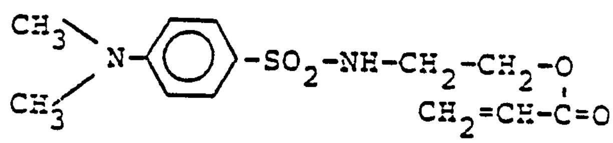 | 89 |
| 10 (US-PS 3 337 592, Beispiel 2) | 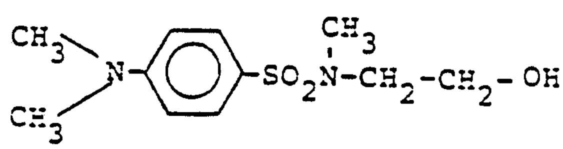 | 90 |
| 11 (C.A. <u>69</u>, 96122 <u>q</u>) | 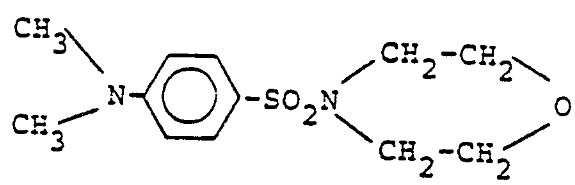 | 202-204 |
| 12 | 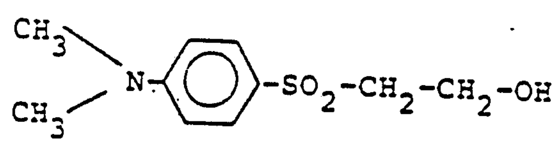 | 102-103 |

**Tabelle I (Fortsetzung)**

| Verbindung Nr. | Formel | Fp. (°C) |
|---|---|---|
| 13 | 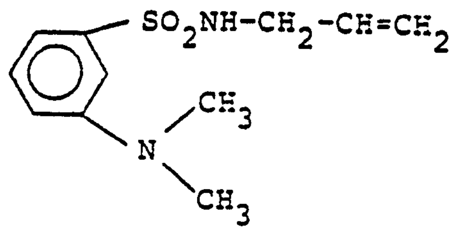 | 71-72 |
| 14 | 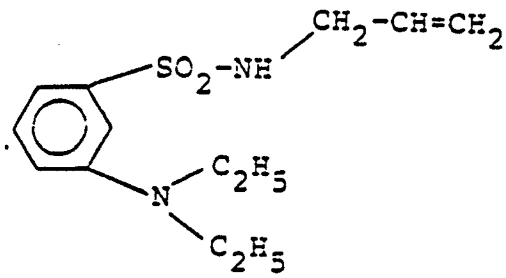 | 60-61 |
| 15 | 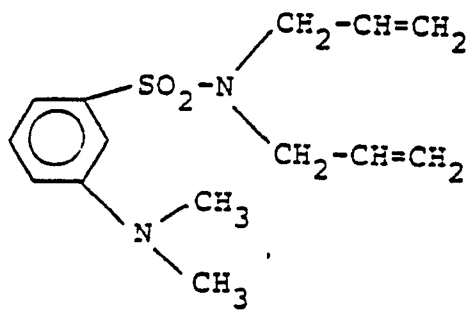 | 23-24 |
| 16 | 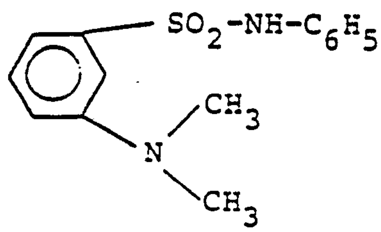 | 115-117 |
| 17 | 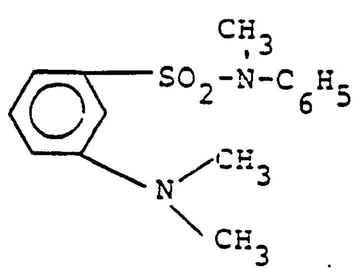 | 137-144 |

**Tabelle I (Fortsetzung)**

| Verbindung Nr. | Formel | Fp. (°C) |
|---|---|---|
| 18 | 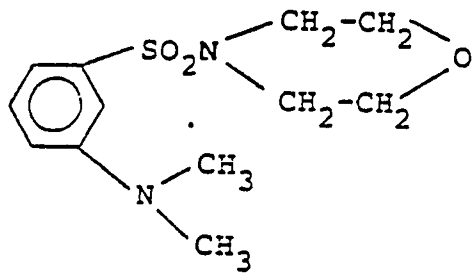 | 123-124 |
| 19 | 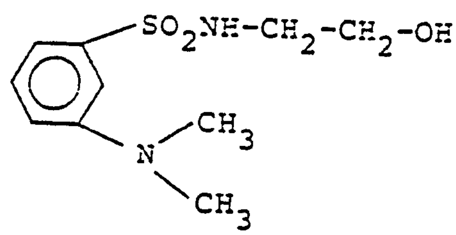 | 99-100 |
| 20 | 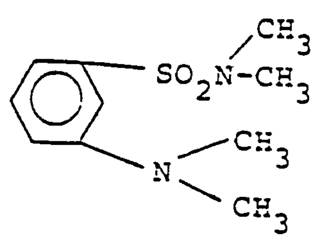 | 53-54 |
| 21 | 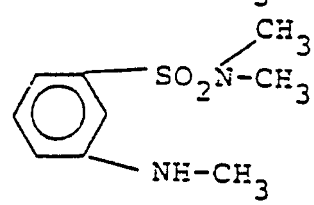 | 81 |
| 22 | 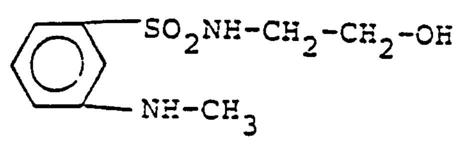 | 128-130 |
| 23 (DE-PS 737 796) | 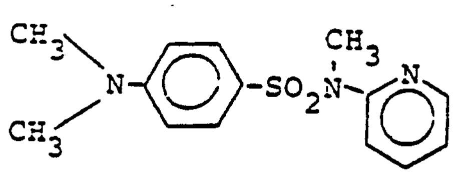 | 155 |

Die in den erfindungsgemäßen Massen enthaltenen Monomeren weisen mindestens eine radikalisch polymerisierbare Doppelbindung auf. Vorzugsweise werden Monomere mit mehr als einer Doppelbindung und Siedepunkten über 100°C bei 13 mbar verwendet. Dadurch werden hochvernetzte Polymerisate oder Copolymerisate erhalten. Die Molgewichte der Monomeren können zwischen etwa 70 und 20000 liegen, vorzugsweise zwischen etwa 150 und 1000. Die Viskosität der Monomeren kann durch geeignete Abmischung von höherviskosen bzw. höhermolekularen Monomeren mit niedrigviskosen Monomeren eingestellt werden. Für die Einstellung der Transparenz von hochgefüllten Dentalmassen gemäß US 3 066 112 ist es erforderlich, die Brechzahl der Polymeren der Brechzahl der Füllstoffe anzupassen. Die Monomeren enthalten gegebenenfalls geringe Mengen an Polymerisationsinhibitoren, wie z.B. 0,01-0,2 % 2,6-Di-t-butyl-p-kresol.

Als Monomere kommen beispielsweise in Frage:

Ester von ungesättigten Mono- oder Dicarbonsäuren, z.B. Ester der Acryl-, Methacryl-, α-Cyanacryl-, Croton-, Zimt-, Sorbin-, Malein-, Fumar- oder Itaconsäure mit aliphatischen, cycloaliphatischen oder aromatischaliphatischen ein- bis vierwertigen Alkoholen mit 2-30 Kohlenstoffatomen, z.B. Methyl(meth-)acrylat, n-, i- und t-Butyl (meth-)acrylat, 2-Ethylhexylacrylat, Laurylacrylat, Dihydrodicyclopentadienyl-(meth-)acrylat, Dihydroxymethyl-tricyclo[5,2,1,$0^{2,6}$]decan-di(meth)-acrylat gemäß der DE-PS 2 200 021, Methylglycoldi(meth)acrylat, Hydroxyethyl(meth)acrylat, Hydroxypropyl (meth)acrylat, Ethylenglykoldiacrylat, Diethylenglykoldiacrylat, Triethylenglykoldi(meth)acrylat, Neopentylglykoldi(meth)acrylat, 1,4-Dimethylolcyclohexandiacrylat, Pentaerythrit-tri- und tetra(meth)acrylat, Trimethylolpropan-tri(meth)acrylat, Ethyl-α-cyanacrylat, Ethylcrotonat, Ethylsorbinat, Diethylmaleinat, Diethylfumarat sowie das Di(meth)acrylat des oxalkylierten Bisphenol A gemäß US-PSS 3 810 938 und 3 923 740, Di(meth)acrylsäureester von oxalkyliertem Trimethylolpropan oder Pentaerythrit gemäß US-PS 3 380 831 und auch die (Meth)acrylester von oxyalkylierten Di-(hydroxy-methyl)-tricyclo[5,2,1,$0^{2,6}$]-decanen, wie sie in DE-OS 2 931 925 und DE-OS 2 931 926 beschrieben werden.

Weitere erfindungsgemäß einsetzbare Monomere sind Amide der (Meth)acrylsäure, die gegebenenfalls am Stickstoffatom mit Alkyl-, Alkoxyalkyl- oder Hydroxyalkylresten substituiert sein können, wie z.B. N-Isobutylacrylamid, Diacetonacrylamid, N-Methylolacrylamid, N-Methoxymethylacrylamid, N-Butoxymethylmethacrylamid, Ethylenglykolbis-(N-methylolacrylamid)-ether und Methylen-bis-acrylamid; Triacrylformal; Vinylester von Mono- und Dicarbonsäuren mit 2 bis 20 Kohlenstoffatomen, z.B. Vinylacetat, Vinylpropionat, 2-Ethylhexansäurevinylester, Versaticsäurevinylester und Divinyladipat; Vinylether von ein- oder zweiwertigen Alkoholen mit 3 bis 20 Kohlenstoffatomen, z.B. Isobutylvinylether, Octadecylvinylether, Ethylenglykoldivinylether und Diethylenglykoldivinylether; Mono-N-Vinylverbindungen, z.B. N-Vinylpyrrolidon, N-Vinylpiperidon, N-Vinylcaprolactam, N-Vinylmorpholin, N-Vinyloxazolidon, N-Vinylsuccinimid, N-Methyl-N-vinylformamid und N-Vinylcarbazol; Allylether und -ester, z.B. Trimethylolpropandiallylether, Trimethylolpropantriallylether, Allyl(meth-)acrylat, Diallylmaleinat, Diallylphthalat und dessen Präpolymere; ungesättigte Polyester mit Molgewichten zwischen 500 und 10 000, gegebenenfalls verdünnt mit Styrol oder Acrylsäureestern.

Zur Herstellung von photopolymerisierbaren Dentalmassen sind von besonderer Wichtigkeit die Epoxidacrylate und Urethanacrylate. Als Beispiele für solche Verbindungen seien aufgeführt:

a) Reaktionsprodukte aus monofunktionellen Epoxiden und (Meth)acrylsäuren gemäß US-PS 2 484 487 und US-PS 2 575 440;

b) Reaktionsprodukte aus bifunktionellen Epoxiden und ungesättigten Fettsäuren nach US-PS 2 456 408;

c) Reaktionsprodukte aus polyfunktionellen aromatischen oder aliphatischen Glycidethern und (Meth)acrylsäure gemäß US-PSS 3 179 623, 3 066 112, 2 824 851 und DE-PS 1 644 817;

d) Reaktionsprodukte aus Epoxidharzen und (Meth)acrylsäurechlorid gemäß US-PS 3 427 161 und US-PS 2 890 202;

e) ungesättigte Polyurethane (Urethanacrylate) und Polyharnstoffe aus Hydroxyalkyl(meth)acrylaten, Aminoalkyl(meth)acrylaten und gegebenenfalls Polyolen oder Polyaminen, wie sie z.B. in den US-PSS 3 425 988, 3 709 866, 3 629 187, 4 089 763 und 4 110 184 sowie den DE-PSS 1 644 798, 1 644 797,DOS 2 357 402, 2 357 324, 2 358 948 beschrieben sind.

Weitere Beispiele geeigneter Comonomere sind der nachstehenden Zusammenstellung zu entnehmen; in den Strukturformeln stehen

R für

$$CH_2=\underset{CH_3}{\underset{|}{C}}-CO-$$

oder $CH_2=CH\text{-}CO$

R' für H oder $-CH_2\text{-}OR$

n für eine ganze Zahl zwischen 1 und 4 und

m für eine ganze Zahl zwischen 0 und 4

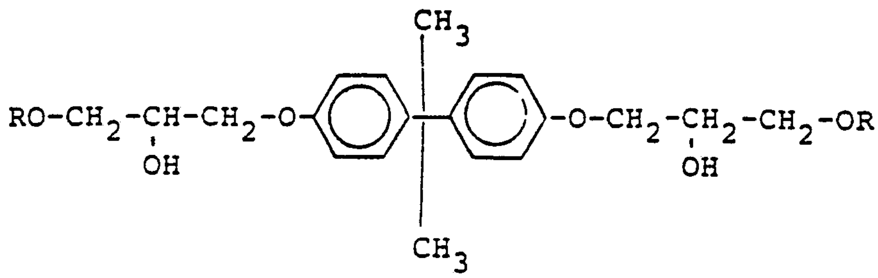

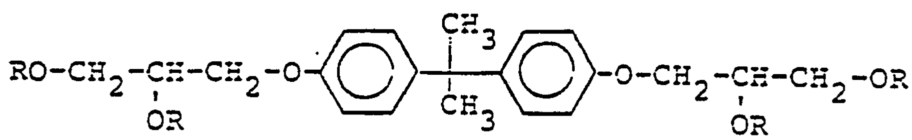

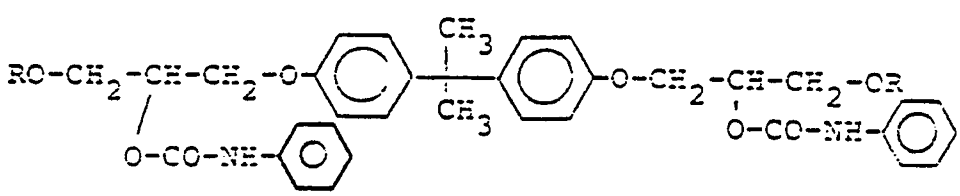

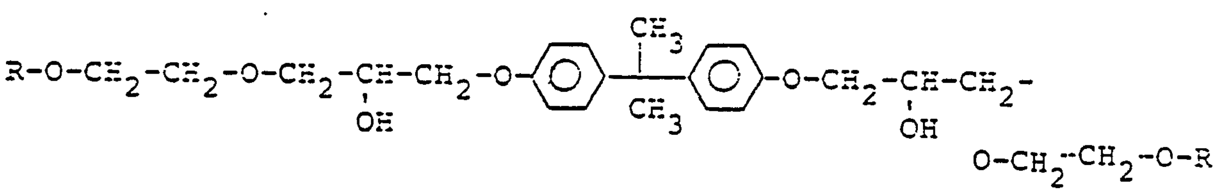

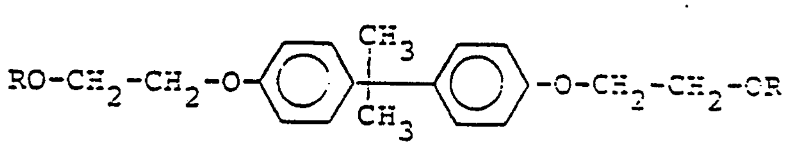

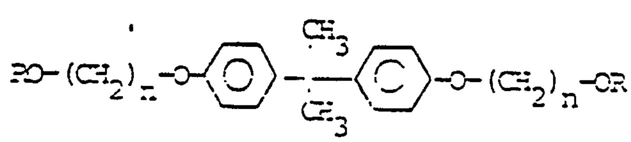

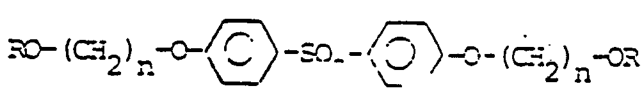

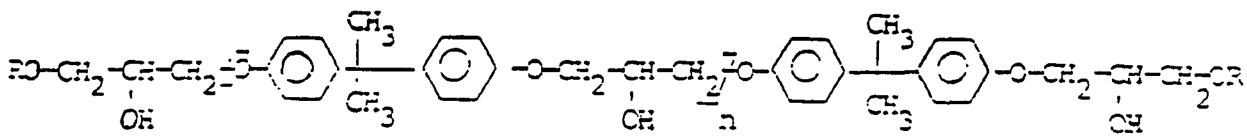

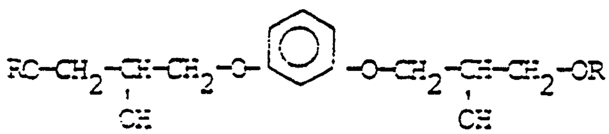

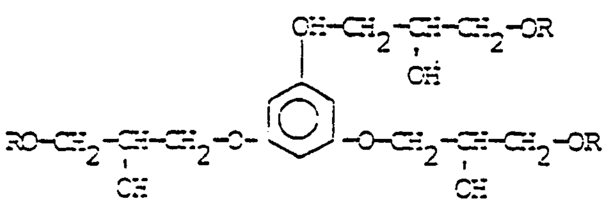

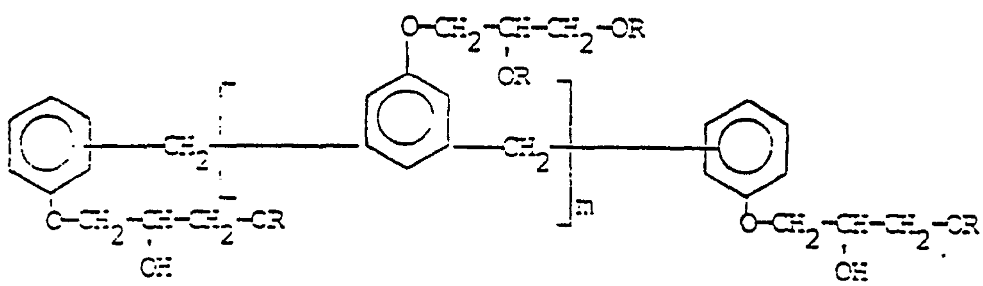

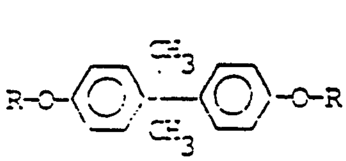

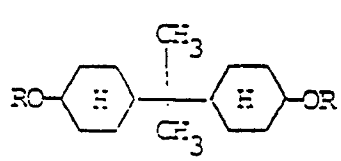

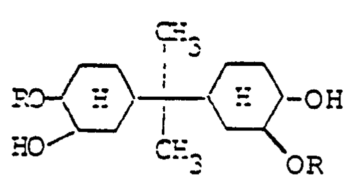

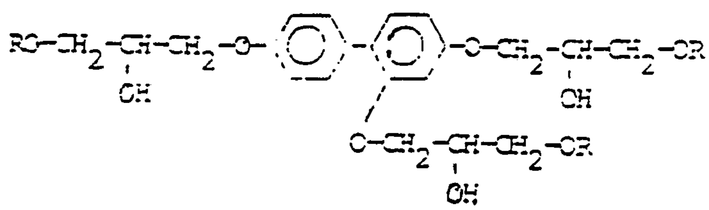

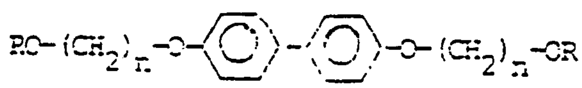

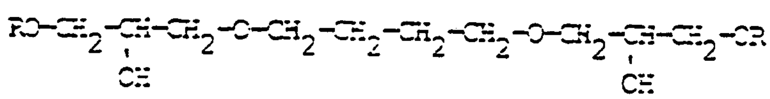

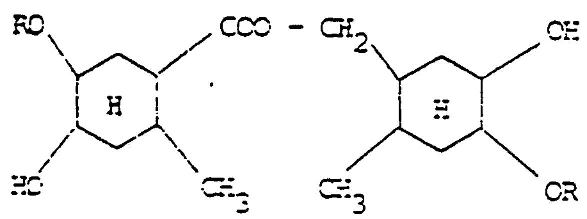

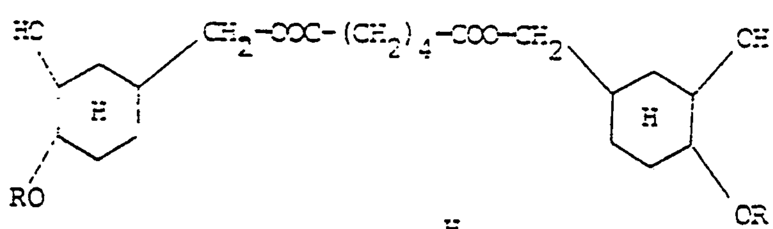

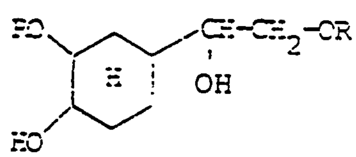

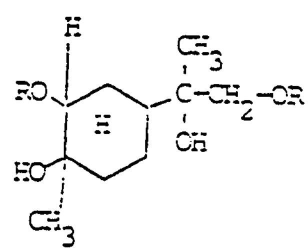

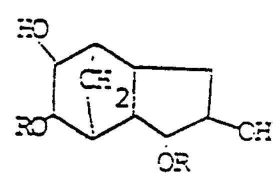

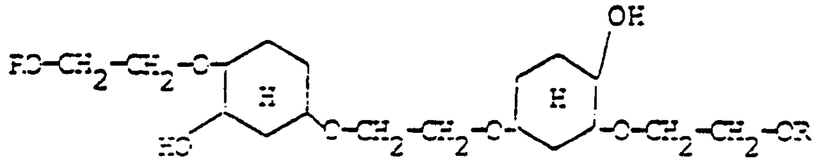

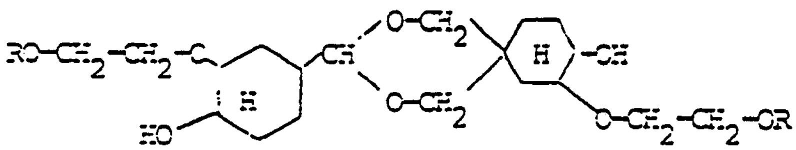

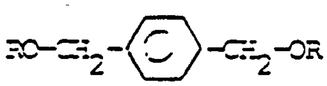

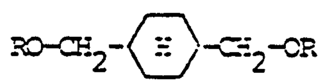

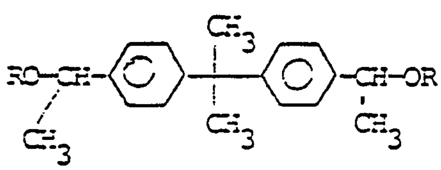

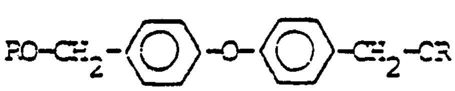

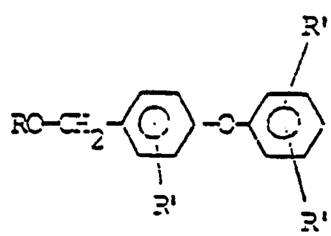

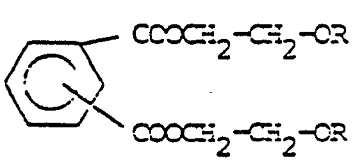

in der ortho-, meta- oder para-Form

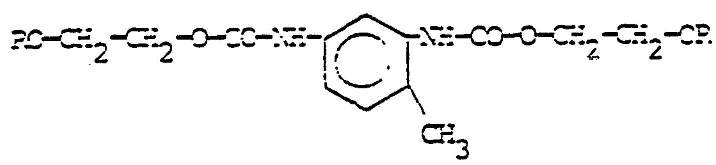

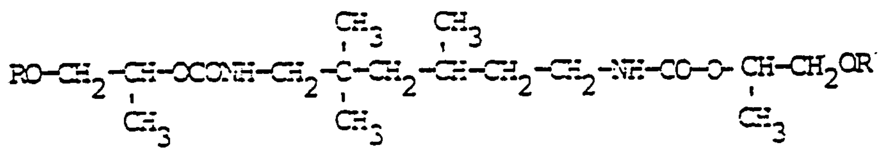

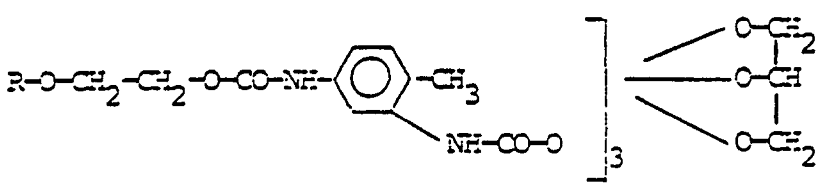

sowie Verbindungen der allgemeinen Formel

$R-(O-D-O-X)_n-OD-OR$

wobei HO-D-OH ein Polyol und HO-X-OH eine Dicarbonsäure darstellen, die jeweils gesättigt oder ungesättigt, cyclisch oder acyclisch sein können.

Je nach Anwendungszweck können den erfindungsgemäßen photopolymerisierbaren Kompositionen aus Monomeren, Photoinitiatoren und -aktivatoren auch noch andere Stoffe zugemischt werden, wie z.B. anorganische und/oder organische Füllstoffe und Pigmente, Stabilisatoren, Farbstoffe, spezielle Lichtschutzmittel, Fluoreszenzmittel, Weichmacher sowie lösliche, quellbare oder unlösliche hochmolekulare Verbindungen.

Insbesondere bei der Verwendung der erfindungsgemäßen photopolymerisierbaren Kompositionen auf dem Dentalgebiet, beispielsweise für die Herstellung von Zahnreparaturmaterialien, Zahnersatzteilen und Versiegelungsmassen ist eine niedrigviskose bis pastöse Konsistenz der Mischungen aus photoaktivierten Monomeren und feinteiligen Füllstoffen erwünscht, wobei trotz des Einsatzes von anorganischen und/oder organischen Füllstoffen eine ausreichende Polymerisationstiefe verlangt wird. Die Teilchengröße der Füllstoffe kann z.B. zwischen 10 nm und ca. 50 µm liegen.

Vorzugsweise beträgt die Menge der Füllstoffe 10 bis 85 Gew.-%, insbesondere 40 bis 80 Gew.-%, bezogen auf photopolymerisierbare Monomere. In manchen Fällen, z.B. bei der Verwendung als Versiegelungsmassen, ist ein Füllstoffzusatz nicht unbedingt erforderlich.

Als anorganische Füllstoffe kommen z.B. Metall-Oxide, Silikate, Phosphate, Sulfate, Carbonate und Fluoride in Frage. Im einzelnen seien aufgeführt: Bergkristall, Quarzit, Novakulit, Cristobalit, Quarzglas, hochdisperse Kieselsäure, Aluminiumoxid, Zirkondioxid, Titandioxid, Bariumsulfat, Calciumfluorid, Barium- oder Calciumsilikate, β-Eukryptit, Spodumen, Borsilikatgläser, Glaskeramiken, z.B. auf der Basis von µ-Cordierit sowie Lanthan und Zirkon enthaltende Glaskeramiken gemäß DE-OS 2 347 591.

Die anorganischen Füllstoffe werden vorzugsweise in an sich bekannter Weise mit einem Haftvermittler vorbehandelt, um den Verbund zur Polymermatrix zu erhöhen. Hierzu sind besonders Silane wie Trimethoxy-(3-methacrylyloxypropyl)-silan oder Titansäureester geeignet.

Als organische feinteilige Füllstoffe werden z.B. Polymere verwendet, welche durch Polymerisation von Vinylmonomeren, Pfropfpolymerisation, Polyaddition oder Polykondensation hergestellt worden sind. Im allgemeinen werden Vinylpolymerisate eingesetzt, welche durch Masse-, Suspensions-, Emulsions- oder Fällungspolymerisation hergestellt wurden. Vorzugsweise verwendet man für Dentalmassen Suspensions-(perl-)polymerisate oder durch Mahlung erzeugte Splitterpolymerisate. Der Quellungsgrad der Polymerisate kann durch Einpolymerisieren von polyfunktionellen Monomeren herabgesetzt werden.

Als geeignete Polymerisate seien beispielhaft Homo- bzw. Copolymerisate aus (Meth)acrylsäureestern, wie Methylmethacrylat, Ethylacrylat, Isobutylmethacrylat, Dodecylmethacrylat, Ethylenglykoldimethacrylat,

Triethylenglykoldimethacrylat, Bisphenoldiglycidyldimethacrylat und 1,12-Dodecandiol-dimethacrylat genannt. Gut geeignet sind auch Polymerisate, die gemäß DE-OS 28 49 280 erhalten wurden.

Die organischen feinteiligen Füllstoffe können auch anorganische mikrofeine Substanzen in feinverteilter Form enthalten. Die Verwendung solcher Hybridfüllstoffe gibt gewisse Vorteile bezüglich der Polierbarkeit und Abriebfestigkeit der daraus hergestellten Dentalwerkstoffe. Hybridfüllstoffe lassen sich als Splitterpolymerisate erhalten, indem Mischungen aus Monomer und mikrofeinen anorganischen Substanzen in Masse polymerisiert und anschließend durch Mahlung zerkleinert werden. In Perlform können derartige Hybridfüllstoffe gemäß DE-OS 28 49 936 hergestellt werden.

Die nachfolgenden Beispiele dienen zur näheren Erläuterung der vorliegenden Erfindung. Wenn nicht anders vermerkt, sind Mengenangaben als Gewichtsteile bzw. Gewichtsprozente zu verstehen.

Als Lichtquelle wurden handelsübliche Dentallampen mit Lichtleiter verwendet, wie sie für die Photopolymerisation von Zahnfüllungen im Munde verwendet werden. Bei den Lampen S und U handelt es sich um Quecksilberdampf-Niederdrucklampen, bei welchen durch Vorschaltung geeigneter Filter nur die Linien bei 365, 405 und 436 nm verwendet werden. Bei den Lampen E, T und F handelt es sich um Halogenlampen mit Emissionsmaxima bei 444 (E) 462 (T) und 172 (F) nm.

**Beispiel 1**

Zu einer 0,2 %igen Lösung von 2,3-Bornandion (Campherchinon) in einem Gemisch aus 67 Teilen BIS-GMA* und 33 Teilen **TEGDM fügt man die in Tabelle 2 angegebenen Mengen an Photoaktivator (die Strukturformeln der Aktivatoren sind Tabelle 1 zu entnehmen). In einer Kugelmühle stellt man Pasten aus je 25 Teilen des photoaktivierten Monomergemisches und 75 Teilen silanisierter feinteiliger Glaskeramik auf Basis µ-Cordierit her; die Photopolymerisation erfolgt mittels Lampe E (Blaulicht mit geringem UV -Anteil).

**Tabelle 2**

| Verbindung aus Tabelle 1 | %, bez. auf Monomer | Aushärtungstiefe in mm nach 20 sec | Lampe E 40 sec Belichtungszeit |
|---|---|---|---|
| 1 | gesättigt | 7,0 | 7,7 |
| 18 | 1,35 | 7,2 | 8,5 |
| 17 | 1,45 | 7,5 | 8,6 |
| 16 | 1,38 | 6,9 | 8,0 |
| 11 | gesättigt | 7,5 | 8,7 |
| 4 | 1,2 | 7,1 | 8,1 |
| 5 | 1,34 | 6,3 | 7,5 |
| 13 | 1,2 | 6,7 | 8,0 |
| 14 | 1,34 | 5,8 | 7,0 |
| 12 | 1,15 | 7,0 | 8,4 |

*BIS-GMA = 2,2-Propan-bis[3-(4-phenoxy)-1,2-dihydroxy-propan-1-methacrylat]
**TEGDM = Triethylenglykol-dimethacrylat

**Beispiel 2**

In einer 0,2 %igen Lösung von 2,3-Bornandion im Monomergemisch von Beispiel 1 löst man 0,01 % 2-Chlorthioxanthon und die in der Tabelle 3 angegebenen Mengen an Photoaktivator. Die Herstellung der Pasten erfolgt wie im Beispiel 1, die Aushärtung unter Verwendung der Lampen S (UV), T und F (Blaulicht).

**Tabelle 3**

| Verbindung aus Tabelle 1 | %, bezogen auf Monomer | Aushärtungstiefen in mm Lampe S nach 20 sec | 40 sec | Lampe T 20 sec | 40 sec | Lampe F 20 sec Belichtungszeit |
|---|---|---|---|---|---|---|
| 6 | 1,4 | 5,2 | 6,4 | 6,4 | 7,8 | 5,7 |
| 4 | 1,2 | 5,1 | 6,0 | 6,0 | 7,8 | 6,0 |
| 13 | 1,2 | 4,5 | 5,5 | 6,2 | 7,6 | 5,95 |
| 15 | 1,4 | 4,6 | 5,5 | 6,3 | 7,4 | 5,8 |
| 12 | 1,15 | 5,4 | 6,3 | 6,1 | 7,4 | 5,9 |
| 8 | 1,56 | 5,0 | 6,1 | 6,2 | 7,1 | 5,7 |
| 7 | 1,2 | 5,1 | 5,9 | 6,2 | 7,1 | 5,5 |
| 11 | gesättigt | 4,5 | 5,4 | 6,1 | 7,2 | 5,6 |
| 5 | 1,34 | 4,6 | 5,8 | 5,3 | 6,6 | 5,1 |
| 14 | 1,34 | 3,6 | — | 5,7 | — | 4,6 |
| 18 | 1,35 | 4,7 | 5,3 | 6,1 | 6,9 | — |
| 21 | 1,07 | 2,3 | 3,3 | 2,6 | 4,0 | — |
| 20 | 1,14 | 4,0 | 4,7 | 5,6 | 6,8 | — |
| 22 | 1,15 | 2,3 | 3,1 | 2,1 | 3,1 | — |
| 19 | 1,22 | 4,0 | 4,6 | 5,5 | 6,4 | — |
| DMAEM* | 0,8 | 4,9 | 5,9 | 5,8 | 6,8 | 4,8 |

* Dimethylaminoethylmethacrylat, zum Vergleich. Man erkennt, daß gleiche Molmengen des hautreizenden DMAEM vielen erfindungsgemäßen Photoaktivatoren unterlegen sind.

**Beispiel 3**

Man löst im Monomergemisch von Beispiel 1 0,025 % Phenanthrenchinon und
a) 1,56 % der Verbindung 8 (0,5 m Mol/100 g) bzw.
b) 1,5 % Dimethylaminoethylmethylacrylat (0,96 m Mol/100 g), Vergleichsversuch.
Die Herstellung der Pasten erfolgt wie in Beispiel 1 unter Verwendung der Lampen S (UV) und T (Blaulicht).

| Versuch | Verbindung | Aushärtungstiefe in mm Lampe S 20 sec | 40 sec | Lampe T 20 sec | 40 sec |
|---|---|---|---|---|---|
| a | 8 | 3,2 | 4,0 | 3,3 | 4,7 |
| b | DMAEM | 2,7 | 3,6 | 4,1 | 5,4 |

Man erkennt, daß wesentlich größere Molmengen des hautreizenden Dimethylaminoethylmethacrylats erforderlich sind, um etwa die gleichen Aushärtungstiefen zu erreichen.

**Beispiel 4**

Man löst im Monomergemisch von Beispiel 1 0,2 % 2,3-Bornandion und 1,56 % der Verbindung 8 als Photoaktivator, sowie steigende Mengen des UV-Initiators 1-Phenyl-2-hydroxy-2-methyl-butanon-1. Die Herstellung der Pasten erfolgt analog Beispiel 1.

| | Aushärtungstiefen in mm | | | |
|---|---|---|---|---|
| % UV-Initiator bez. auf Monomer | Lampe S 20 sec | 40 sec | Lampe T 20 sec Belichtungszeit | 40 sec |
| 0 | 2,3 | 3,1 | 6,0 | 7,0 |
| 0,25 | 4,1 | 4,9 | 5,3 | 6,2 |
| 0,5 | 4,7 | 5,1 | 5,3 | 6,6 |
| 1,0 | 4,94 | 4,7 | 5,7 | 6,6 |
| 2,0 | 4,55 | 5,0 | 5,6 | 6,4 |
| 4,0 | 4,4 | 5,0 | 5,7 | 6,6 |

**Beispiel 5**

Man löst im Monomergemisch von Beispiel 1 0,025% 1-Benzoylamino-4-chloranthrachinon und setzt steigende Mengen der Verbindung 8 hinzu. Die Herstellung der Pasten erfolgt analog Beispiel 1.

| | Aushärtung in mm | |
|---|---|---|
| % Verbindung 8, bez. auf Monomer | Lampe E 20 sec | 40 sec |
| 0 | 0 | 0 |
| 0,185 | 1,2 | 1,7 |
| 0,39 | 1,5 | 2,3 |
| 0,78 | 1,9 | 3,0 |
| 1,04 | 2,2 | 3,2 |
| 1,56 | 2,6 | 3,4 |

**Beispiel 6**

In einem mit 0,1 % 2,6-Di-t-butyl-p-kresol stabilisierten Monomergemisch aus 67 Teilen BIS-GMA und 33 Teilen TEGDM werden 0,2 % 2,3-Bornandion (Campherchinon), 0,01 % 2-Chlorthioxanthon und die in Tabelle 4 angegebenen Mengen an Photoaktivator gelöst.

Zu 100 g des aktivierten Monomergemisches werden

98 g silanisiertes Siliziumdioxid mit einer BET-Oberfläche von 50 $m^2/g$ (silanisiert mit 7,5 % γ-Methacryloxypropyltrimethoxysilan),

140g Perlpolymerisat mit einer mittleren Teilchengröße von 90 μ, hergestellt gemäß DE-OS 28 49 936 durch Polymerisation von 28 % Siliziumdioxid (BET-Oberfläche: 50 $m^2/g$), 50 % Methylmethacrylat, 18 % Isobutylmethacrylat und 4 % BIS-GMA,

1,1 mg Paliogenrot und

7,2 mg Irgazingelb

gegeben und die Komponenten unter Kühlen in einem Kneter 30 Minuten lang intensiv gemischt, wobei während der letzten 10 Min. ein Vakuum von 13 mbar angelegt wird.

Die Pasten werden mit den Lampen U und T ausgehärtet, es entsteht dabei ein Material mit zahnfarbenem Aussehen.

**Tabelle 4**

| Verbindung | %, bezogen auf Monomer | Aushärttiefen (mm) Lampe U 10 | 30 | 60 sec | Lampe T 10 | 30 | 60 sec | Opazität nach ADA 27 | Diametrale Zugfestigkeit nach ADA 27 (MPa) |
|---|---|---|---|---|---|---|---|---|---|
| DMAEM* | 1,5 | 1,8 | 2,8 | 3,4 | 3,2 | 4,6 | 5,2 | 0,36 | 37,43 |
| 6 | 1,4 | 2,3 | 3,0 | 3,7 | 3,4 | 4,7 | 5,6 | 0,36 | 40,26 |
| 7 | 1,2 | 2,2 | 3,0 | 3,6 | 3,3 | 4,5 | 5,4 | 0,38 | 41,40 |
| 8 | 1,55 | 2,3 | 3,2 | 3,8 | 3,6 | 4,8 | 5,5 | 0,37 | 42,60 |

* Vergleichsbeispiel

**Beispiel 7**

Zur Herstellung einer aktivierten Monomerlösung werden in 100 g des durch Umsetzung von 1 Mol 2,2,4-Trimethylhexamethylen-diisocyanat und 2 Mol 2-Hydroxyethylmethacrylat erhaltenen Urethandimethacrylates 0,01 g 2-Chlorthioxanthon, 0,2 g 2,3-Bornandion (Campherchinon) und 1,2 g 4-(N,N-Dimethylamino-)benzolsulfonoxethylamid (Verbindung 7) gelöst.

Weiterhin werden zur Herstellung eines Splitterpolymerisates eine Mischung aus 120 g des oben beschriebenen Urethandimethacrylates, 20 g Triethylenglykoldimethacrylat und 200 g silanisiertes Siliziumdioxid (BET-Oberfläche 50 $m^2/g$, silanisiert mit 10 % γ-Methacryloxypropyltrimethoxysilan) durch Zugabe von 0,5 g Benzoylperoxid bei 100°C in Masse polymerisiert, und die erhaltene auspolymerisierte Masse in einer Kugelmühle auf eine mittlere Teilchengröße von 40 µm aufgemahlen.

Aus 91 g aktivierter Monomerlösung, 145 g Splitterpolymerisat, 82 g Siliziumdioxid (BET-Oberfläche 50 $m^2/g$, silanisiert mit 10 % γ-Methacryloxypropyltrimethoxysilan), 0,7 mg Paliogenrot und 4,3 g Irgazingelb wird nach der in Beispiel 6 angegebenen Arbeitsweise eine Paste hergestellt.

Die Paste wird mit den Lampen U und T ausgehärtet, es entsteht dabei ein Material mit zahnfarbenem Aussehen.

Aushärttiefen [mm]

| Lampe U 20 | 60 sec | Lampe T 20 | 60 sec | Opazität nach ADA 27 | diametrale Zugfestigkeit nach ADA 27 [MPa] |
|---|---|---|---|---|---|
| 2,5 | 3,8 | 4,5 | 5,5 | 0,39 | 39,80 |

**Beispiel 8**

Zur Herstellung einer aktivierten Monomerlösung werden in 200 g Bis-β-methacryloxy-ethyloxymethyl-tricyclo-[5.2.0.1.$^{2.6}$]-decan

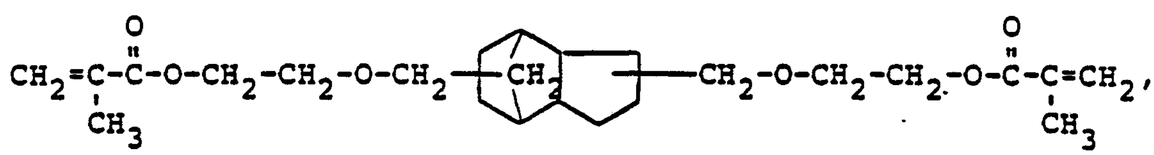

(hergestellt gemäß DE-OS 29 31 925) 0,02 g Chlorthioxanton, 0,4 g 2,3-Bornandion (Campherchinon) und 2,4 g 4-(N,N-Dimethylamino-)benzolsulfonoxethylamid (Verbindung 7) gelöst.

**Paste A**

Aus 97 g aktivierter Monomerlösung, 150 g Siliziumdioxid (BET-Oberfläche 50 $m^2/g$, silanisiert mit 10 % γ-Methacryloxypropyltrimethoxysilan), 0,5 mg Paliogenrot und 3,2 mg Irgazingelb wird nach der in Beispiel 6 angegebenen Arbeitsweise eine Paste hergestellt.

**Paste B**

In entsprechender Weise wird aus 80 g aktiviertem Monomer und 120 g Perlpolymerisat mit einer mittleren Teilchengröße von 40 μ, hergestellt aus 60 % Methylmethacrylat, 30 % Isobutylmethacrylat und 10 % Bis-β-meth-acryloxyethoxymethyl-tricyclo-[5.2.0.1.$^{2.6}$]-decan, 0,65 mg Paliogenrot und 4,1 mg Irgazingelb, eine Paste hergestellt.

Die Pasten A und B werden mit den Lampen U und T ausgehärtet, es entsteht ein Material mit zahnfarbenem Aussehen.

**Aushärttiefen** [mm]

| | Lampe U 20 | Lampe U 60 sec | Lampe T 20 | Lampe T 60 sec | Opazität nach ADA 27 | diametrale Zugfestigkeit nach ADA 27 [MPa] |
|---|---|---|---|---|---|---|
| Paste A | 4,0 | 4,8 | 6,5 | 7,4 | 0,35 | 43,25 |
| Paste B | 3,9 | 4,5 | 5,6 | 7,1 | 0,43 | 35,12 |

**Patentansprüche**

1. Photopolymerisierbare Massen, enthaltend
a) mindestens ein ethylenisch ungesättigtes, photopolymerisierbares Monomer,
b) mindestens einen Photoinitiator aus der Gruppe der organischen Mono- und Dicarbonylverbindungen und
c) mindestens ein aromatisches Amin, das in α-Stellung zum Stickstoff eine CH-Gruppe aufweist, als Photoaktivator, und gegebenenfalls
d) mindestens einen feinteiligen anorganischen und/oder organischen Füllstoff,
dadurch gekennzeichnet, daß die Photoaktivatoren Alkylaminoarylsulfonylverbindungen der allgemeinen Formel

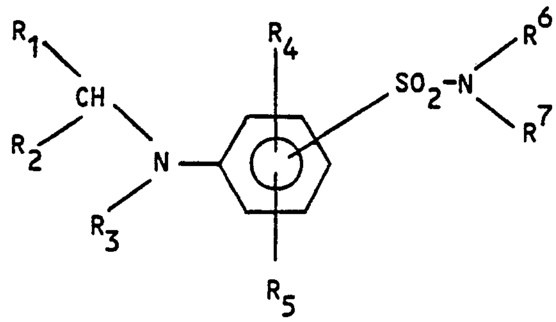

in welcher
$R^1$ und $R^2$ gleich oder verschieden sind und Wasserstoff, Methyl, Vinyl oder Phenyl bedeuten,
$R^3$ Wasserstoff, Methyl und Ethyl bedeutet,
$R^4$ und $R^5$ gleich oder verschieden sind und Wasserstoff, Halogen oder Methyl bedeuten oder orthoständiges $R^4$ und $R^5$ Teile eines annellierten aromatischen Sechsrings sind,
$R^6$ und $R^7$ gleich oder verschieden sind und Wasserstoff, Methyl, Allyl, Methallyl, Cyclohexyl, Phenyl, Benzyl, Hydroxyethyl, Hydroxypropyl, Acryloxyalkyl, Methacryloxyalkyl, 2,3-Epoxypropyl oder 1,2-Dihydroxypropyl-1-(meth-)acrylat bedeuten und wobei $R^6$ und $R^7$ zu einem Morpholin- oder Piperidinrest verbunden sein können, sind.

2. Photopolymerisierbare Masse nach Anspruch 1, dadurch gekennzeichnet, daß das Verhältnis Photoinitiator zu Photoaktivator 0,01 zu 1 bis 10 beträgt.

3. Photopolymerisierbare Masse nach den Ansprüchen und 2, dadurch gekennzeichnet, daß 0,001 bis 10 Gew.-% des Photoinitiators, bezogen auf das ethylenisch ungesättigte Monomer, eingesetzt werden.

4. Verfahren zur Herstellung von photopolymersierbaren Massen, dadurch gekennzeichnet, daß man
a) mindestens ein ethylenisch ungesättigtes, photopolymerisierbares Monomer,
b) mindestens einen Photoinitiator aus der Gruppe der organischen Mono- und Dicarbonylverbindungen und
c) mindestens ein aromatisches Amin, das in α-Stellung zum Stickstoff eine CH-Gruppe aufweist, als Photoaktivator, und gegebenenfalls
d) mindestens einen feinteiligen anorganischen und/oder organischen Füllstoff,
wobei die Photoaktivatoren Alkylaminoarylsulfonylverbindungen der allgemeinen Formel

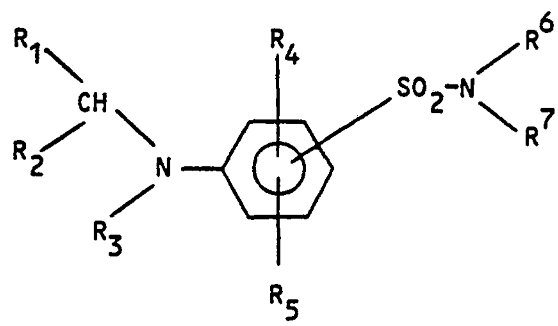

in welcher
$R^1$ und $R^2$ gleich oder verschieden sind und Wasserstoff, Methyl, Vinyl oder Phenyl bedeuten,
$R^3$ Wasserstoff, Methyl und Ethyl bedeutet,
$R^4$ und $R^5$ gleich oder verschieden sind und Wasserstoff, Halogen oder Methyl bedeuten oder orthoständiges $R^4$ und $R^5$ Teile eines annellierten aromatischen Sechsrings sind,
$R^6$ und $R^7$ gleich oder verschieden sind und Wasserstoff, Methyl, Allyl, Methallyl, Cyclohexyl, Phenyl, Benzyl, Hydroxyethyl, Hydroxypropyl, Acryloxyalkyl, Methacryloxyalkyl, 2,3-Epoxypropyl oder 1,2-Dihydroxypropyl-1-(meth-)acrylat bedeuten und wobei $R^6$ und $R^7$ zu einem Morpholin- oder Piperidinrest verbunden sein können, sind,

intensiv mischt.

5. Verwendung von photopolymerisierbaren Massen, enthaltend

a) mindestens ein ethylenisch ungesättigtes, photopolymerisierbares Monomer,

b) mindestens einen Photoinitiator aus der Gruppe der organischen Mono- und Dicarbonylverbindungen und

c) mindestens ein aromatisches Amin, das in α-Stellung zum Stickstoff eine CH-Gruppe aufweist, als Photoaktivator, und gegebenenfalls

d) mindestens einen feinteiligen anorganischen und/oder organischen Füllstoff,

wobei

Alkylaminoarylsulfonylverbindungen der allgemeinen Formel

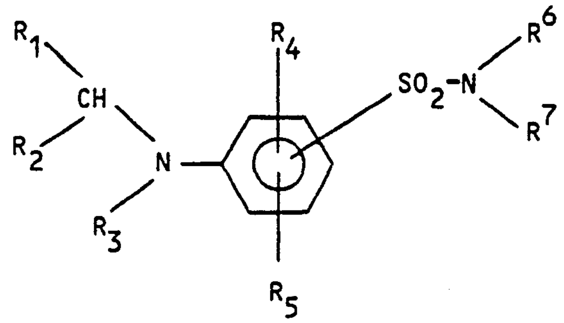

in welcher

$R^1$ und $R^2$ gleich oder verschieden sind und Wasserstoff, Methyl, Vinyl oder Phenyl bedeuten,

$R^3$ Wasserstoff, Methyl und Ethyl bedeutet,

$R^4$ und $R^5$ gleich oder verschieden sind und Wasserstoff, Halogen oder Methyl bedeuten oder orthoständiges $R^4$ und $R^5$ Teile eines annellierten aromatischen Sechsrings sind,

$R^6$ und $R^7$ gleich oder verschieden sind und Wasserstoff, Methyl, Allyl, Methallyl, Cyclohexyl, Phenyl, Benzyl, Hydroxyethyl, Hydroxypropyl, Acryloxyalkyl, Methacryloxyalkyl, 2,3-Epoxypropyl oder 1,2-Dihydroxypropyl-1-(meth-)acrylat bedeuten und wobei $R^6$ und $R^7$ zu einem Morpholin- oder Piperidinrest verbunden sein können,

sind,

im Dentalbereich.

6. Verfahren zur Herstellung von Zahnersatzteilen, Zahnfüllungen oder Überzügen auf Zähnen, dadurch gekennzeichnet, daß man photopolymerisierbare Massen, enthaltend

a) mindestens ein ethylenisch ungesättigtes, photopolymerisierbares Monomer,

b) mindestens einen Photoinitiator aus der Gruppe der organischen Mono- und Dicarbonylverbindungen und

c) mindestens ein aromatisches Amin, das in α-Stellung zum Stickstoff eine CH-Gruppe aufweist, als Photoaktivator, und gegebenenfalls

d) mindestens einen feinteiligen anorganischen und/oder organischen Füllstoff,

dadurch gekennzeichnet, daß die Photoaktivatoren Alkylaminoarylsulfonylverbindungen der allgemeinen Formel

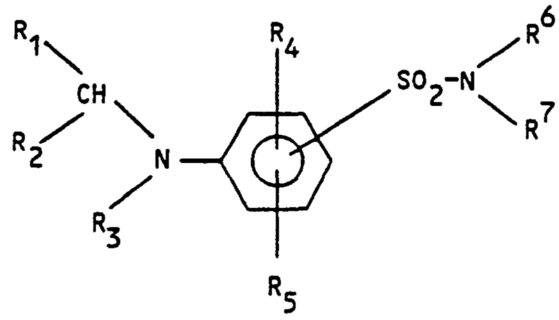

in welcher

$R^1$ und $R^2$ gleich oder verschieden sind und Wasserstoff, Methyl, Vinyl oder Phenyl bedeuten,

$R^3$ Wasserstoff, Methyl und Ethyl bedeutet,

$R^4$ und $R^5$ gleich oder verschieden sind und Wasserstoff, Halogen oder Methyl bedeuten oder orthoständiges $R^4$ und $R^5$ Teile eines annellierten aromatischen Sechsrings sind,

$R^6$ und $R^7$ gleich oder verschieden sind und Wasserstoff, Methyl, Allyl, Methallyl, Cyclohexyl, Phenyl, Benzyl, Hydroxyethyl, Hydroxypropyl, Acryloxyalkyl, Methacryloxyalkyl, 2,3-Epoxypropyl oder 1,2-Dihydroxypropyl-1-(meth-)acrylat bedeuten und wobei $R^6$ und $R^7$ zu einem Morpholin- oder Piperidinrest verbunden sein können,

sind

unter Lichteinwirkung polymerisiert.

**Claims**

1. Photopolymerisable compositions containing
a) at least one ethylenically unsaturated, photopolymerisable monomer,
b) at least one photoinitiator selected from organic mono- and dicarbonyl compounds and
c) at least one aromatic amine which contains a CH group in the α-position to the nitrogen, as the photoactivator, and, optionally,
d) at least one finely divided inorganic and/or organic filler,
characterised in that the photoactivators are alkylaminoarylsulphonyl compounds of the general formula

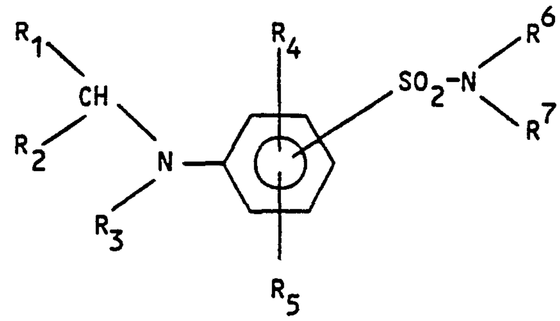

in which
$R^1$ and $R^2$ are identical or different and denote hydrogen, methyl, vinyl or phenyl,
$R^3$ denotes hydrogen, methyl and ethyl,
$R^4$ and $R^5$ are identical or different and denote hydrogen, halogen or methyl or ortho-positioned $R^4$ and $R^5$ are parts of a fused aromatic six-membered ring,
$R^6$ and $R^7$ are identical or different and denote hydrogen, methyl, allyl, methallyl, cyclohexyl, phenyl, benzyl, hydroxyethyl, hydroxypropyl, acryloxyalkyl, methacryloxyalkyl, 2,3-epoxypropyl or 1,2-dihydroxypropyl-1-(meth-) acrylate and it being possible for $R^6$ and $R^7$ to be linked to form a morpholine or piperidine radical.

2. Photopolymerisable composition according to Claim 1, characterised in that the ratio between photoinitiator and photoactivator is 0.01 to 1 to 10.

3. Photopolymerisable composition according to Claims 1 and 2, characterised in that 0.001 to 10% by weight of the photoinitiator, based on the ethylenically unsaturated monomer, are used.

4. Process for the production of photopolymerisable compositions, characterised in that
a) at least one ethylenically unsaturated photopolymerisable monomer,
b) at least one photoinitiator selected from organic mono- and dicarbonyl compounds and
c) at least one aromatic amine which contains a CH group in the α-position to the nitrogen, as the photoactivator, and, optionally
d) at least one finely-divided inorganic and/or organic filler,
the photoactivators being alkylaminoarylsulphonyl compounds of the general formula

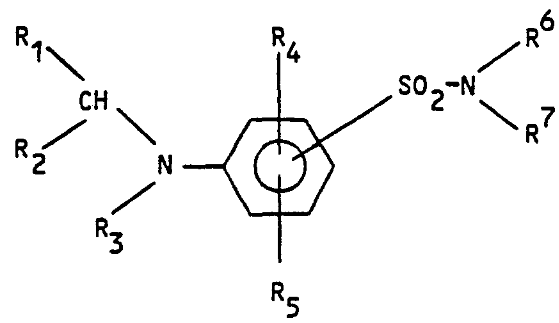

in which
$R^1$ and $R^2$ are identical or different and denote hydrogen, methyl, vinyl or phenyl,
$R^3$ denotes hydrogen, methyl and ethyl,
$R^4$ and $R^5$ are identical or different and denote hydrogen, halogen or methyl or orthopositioned $R^4$ and $R^5$ are parts of a fused aromatic six-membered ring,

$R^6$ and $R^7$ are identical or different and denote hydrogen, methyl, allyl, methallyl, cyclohexyl, phenyl, benzyl, hydroxyethyl, hydroxypropyl, acryloxyalkyl, methacryloxyalkyl, 2,3-epoxypropyl or 1,2-dihydroxypropyl-1-(meth-)acrylate and it being possible for $R^6$ and $R^7$ to be linked to form a morpholine or piperidine radical,

are intensively mixed.

5. Use of photopolymerisable compositions containing

a) at least one ethylenically unsaturated photopolymerisable monomer,

b) at least one photoinitiator selected from organic mono- and dicarbonyl compounds and

c) at least one aromatic amine which contains a CH group in the α-position to the nitrogen, as the photoactivator, and, optionally,

d) at least one finely-divided inorganic and/or organic filler,

wherein

alkylaminoarylsulphonyl compounds are of the general formula

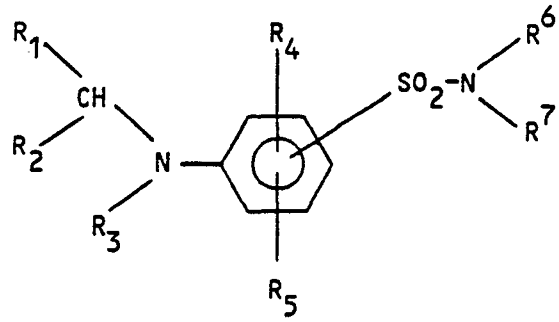

in which

$R^1$ and $R^2$ are identical or different and denote hydrogen, methyl, vinyl or phenyl,

$R^3$ denotes hydrogen, methyl and ethyl,

$R^4$ and $R^5$ are identical or different and denote hydrogen, halogen or methyl or ortho-positioned $R^4$ and $R^5$ are parts of a fused aromatic six-membered ring,

$R^6$ and $R^7$ are identical or different and denote hydrogen, methyl, allyl, methallyl, cyclohexyl, phenyl, benzyl, hydroxyethyl, hydroxypropyl, acryloxyalkyl, methacryloxyalkyl, 2,3-epoxypropyl or 1,2-dihydroxypropyl-1-(meth-)acrylate and it being possible for $R^6$ and $R^7$ to be linked to form a morpholine or piperidine radical,

in the dental field.

6. Process for the production of dental prostheses, dental fillings or coatings on teeth, characterised in that photopolymerisable compositions containing

a) at least one ethylenically unsaturated, photopolymerisable monomer,

b) at least one photoinitiator selected from organic mono- and dicarbonyl compounds and

c) at least one aromatic amine which contains a CH group in the α-position to the nitrogen, as the photoactivator, and, optionally,

d) at least one finely divided inorganic and/or organic filler,

characterised in that the photoactivators are alkylaminoarylsulphonyl compounds of the general formula

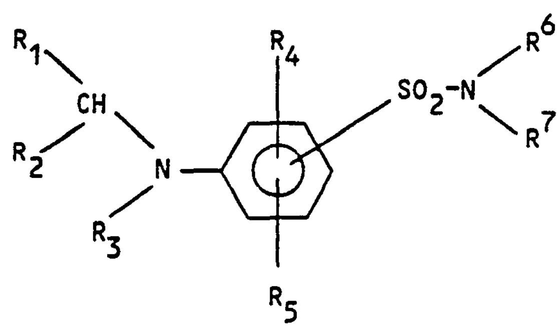

in which

$R^1$ and $R^2$ are identical or different and denote hydrogen, methyl, vinyl or phenyl,

$R^3$ denotes hydrogen, methyl and ethyl,

$R^4$ and $R^5$ are identical or different and denote hydrogen, halogen or methyl or ortho-positioned $R^4$ and $R^5$ are parts of a fused aromatic six-membered ring,

$R^6$ and $R^7$ are identical or different and denote hydrogen, methyl, allyl, methallyl, cyclohexyl, phenyl, benzyl, hydroxyethyl, hydroxypropyl, acryloxyalkyl, methacryloxyalkyl, 2,3-epoxypropyl or 1,2-dihydroxypropyl-1-(meth-)acrylate and it being possible for $R^6$ and $R^7$ to be linked to form a morpholine or piperidine radical,

are polymerised under the action of light.

**Revendications**

1. Masses photopolymérisables contenant:

a) au moins un monomère à insaturation éthylénique photopolymérisable,

b) au moins un photo-inducteur du groupe des composés organiques mono- et di-carbonylés,

c) au moins une amine aromatique portant en position alpha de l'azote, un groupe CH et servant de photo-activateur, et le cas échéant

d) au moins une matière de charge minérale et/ou organique en fines particules,

caractérisées en ce que les photo-activateurs sont des composés alkylaminoarylsulfonyliques de formule générale

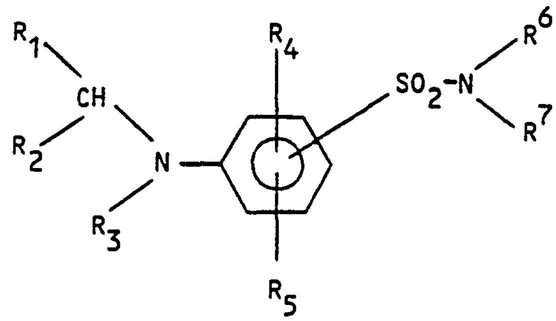

dans laquelle

$R^1$ et $R^2$, ayant des significations identiques ou différentes, représentent chacun l'hydrogène, un groupe méthyle, vinyle ou phényle,

$R^3$ représente l'hydrogène, un groupe méthyle ou éthyle,

$R^4$ et $R^5$, ayant des significations identiques ou différentes, représentent chacun l'hydrogène, un halogène ou un groupe méthyle, ou bien $R^4$ et $R^5$ en position ortho représentent des parties d'un noyau aromatique hexagonal condensé,

$R^6$ et $R^7$, ayant des significations identiques ou différentes, représentent chacun l'hydrogène, un groupe méthyle, allyle, méthallyle, cyclohexyle, phényle, benzyle, hydroxyéthyle, hydroxypropyle, acryloxyalkyle, méthacryloxyalkyle, 2,3-époxypropyle ou 1,2-dihydroxypropyl-1-(méth)acrylate, $R^6$ et $R^7$ pouvant également être reliés entre eux avec formation d'un reste de morpholine ou de pipéridine.

2. Masse photopolymérisable selon la revendication 1, caractérisée en ce que le rapport entre le photo-inducteur et le photo-activateur va de 0,01-1 à 10.

3. Masse photopolymérisable selon les revendications 1 et 2, caractérisée en ce que l'on utilise de 0,001 à 10 % en poids du photo-inducteur par rapport au monomère à insaturation éthylénique.

4. Procédé de préparation de masses photopolymérisables, caractérisé en ce que l'on soumet à mélange intensif:

a) au moins un monomère à insaturation éthylénique photopolymérisable,

b) au moins un photo-inducteur du groupe des composés organiques mono- et di-carbonylés, et

c) au moins une amine aromatique portant un groupe CH en position alpha de l'azote et servant de photo-activateur, et le cas échéant

d) au moins une matière de charge minérale et/ou organique en fines particules,

les photo-activateurs consistant en composés alkylaminoarylsulfonyliques de formule générale

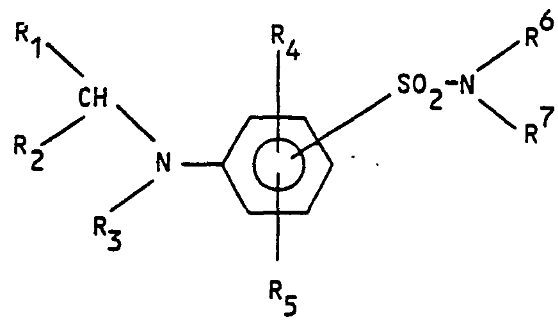

dans laquelle

$R^1$ et $R^2$, ayant des significations identiques ou différentes, représentent chacun l'hydrogène, un groupe méthyle, vinyle ou phényle,

$R^3$ représente l'hydrogène, un groupe méthyle ou éthyle,

$R^4$ et $R^5$, ayant des significations identiques ou différentes, représentent chacun l'hydrogène, un halogène ou un groupe méthyle, ou bien $R^4$ et $R^5$ en position ortho constituent des parties d'un noyau aromatique hexagonal condensé,

$R^6$ et $R^7$, ayant les significations identiques ou différentes, représentent chacun l'hydrogène, un groupe méthyle, allyle, méthallyle, cyclohexyle, phényle, benzyle, hydroxyéthyle, hydroxypropyle, acryloxyalkyle, méthacryloxyalkyle, 2,3-époxypropyle ou 1,2-dihydroxypropyl-1-(méth)acrylate, $R^6$ et $R^7$ pouvant également être reliés entre eux avec formation d'un reste de morpholine ou de pipéridine.

5. Utilisation de masses photopolymérisables contenant:

a) au moins un monomère à insaturation éthylénique photopolymérisable,

b) au moins un photo-inducteur du groupe des composés organiques mono- et di-carbonylés, et

c) au moins une amine aromatique portant un groupe CH en position alpha de l'azote et servant de photo-activateur, et le cas échéant

d) au moins une matière de charge minérale et/ou organique en fines particules des composés alkylaminoarylsulfonyliques de formule générale

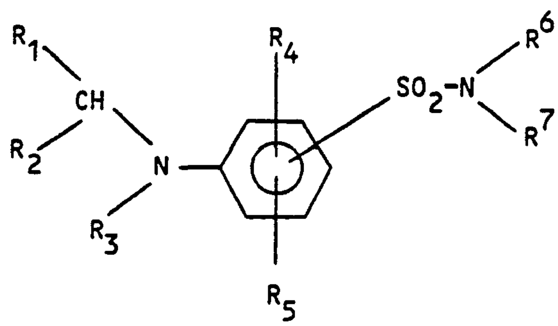

dans laquelle

$R^1$ et $R^2$, ayant des significations identiques ou différentes, représentent chacun l'hydrogène un groupe méthyle, vinyle ou phényle,

$R^3$ représente l'hydrogène, un groupe méthyle ou éthyle,

$R^4$ et $R^5$, ayant des significations identiques ou différentes, représentent chacun l'hydrogène, un halogène ou un groupe méthyle ou bien $R^4$ et $R^5$, en position ortho, constituent des parties d'un noyau aromatique hexagonal condensé,

$R^6$ et $R^7$, ayant des significations identiques ou différentes, représentent chacun l'hydrogène, un groupe méthyle, allyle, méthallyle, cyclohexyle, phényle, benzyle, hydroxyéthyle, hydroxypropyle, acryloxyalkyle, méthacryloxyalkyle, 2,3-époxypropyle ou 1,2-dihydroxypropyl-1-(méth)acrylate, $R^6$ et $R^7$ pouvant également être reliés entre eux avec formation d'un reste de morpholine ou de pipéridine,

dans le domaine dentaire.

6. Procédé pour la préparation de prothèses dentaires, la réalisation d'obturations dentaires ou l'application de revêtements sur les dents, caractérisé en ce que l'on polymérise sous l'action de la lumière des masses photopolymérisables contenant:

a) au moins un monomère à insaturation éthylénique photopolymérisable,

b) au moins un photo-inducteur du groupe des composés organiques mono- et di-carbonylés, et

c) au moins une amine aromatique portant un groupe CH en position alpha de l'azote et servant de photo-activateur, et le cas échéant

d) au moins une matière de charge minérale et/ou organique en fines particules,

caractérisé en ce que les photo-activateurs sont des composés alkylaminoarylsulfonyliques de formule générale

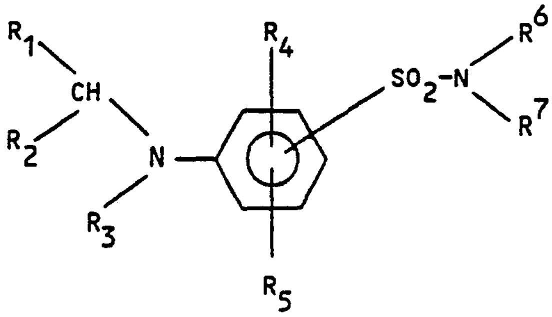

dans laquelle

$R^1$ et $R^2$, ayant des significations identiques ou différentes, représentent chacun l'hydrogène, un groupe méthyle, vinyle ou phényle,

$R^3$ représente l'hydrogène, un groupe méthyle ou éthyle,

$R^4$ et $R^5$, ayant des significations identiques ou différentes, représentent chacun l'hydrogène, un halogène ou un groupe méthyle ou bien $R^4$ et $R^5$, en position ortho, constituent des parties d'un noyau aromatique hexagonal condensé,

$R^6$ et $R^7$, ayant des significations identiques ou différentes, représentent chacun l'hydrogène, un groupe méthyle, allyle, methallyle, cyclohexyle, phényle, benzyle, hydroxyéthyle, hydroxypropyle, acryloxyalkyle, méthacryloxyalkyle, 2,3-époxypropyle ou ou 1,2-dihydroxypropyl-1-(méth)acrylate, $R^6$ et $R^7$ pouvant également être reliés entre eux avec formation d'un reste de morpholine ou de pipéridine.